# EUROPEAN PATENT APPLICATION

(11) **EP 3 460 804 A1**
(43) Date of publication of application: **27.03.2019**
(21) Application number: 17192137.2
(22) Date of filing: 20.09.2017
(51) Int. Cl.: G16H 30/40, G16H 10/60, G16H 30/20, G16H 70/20

(54) **PROVIDING SUBJECT-SPECIFIC INFORMATION**

(71) Applicant: Koninklijke Philips N.V., 5656 AE Eindhoven (NL)
(72) Inventor: SERLIE, Iwo Willem Oscar, 5656 AE Eindhoven (NL); ALGRA, Egbert, 5656 AE Eindhoven (NL); PEETERS, Bob, 5656 AE Eindhoven (NL)
(74) Representative: de Haan, Poul Erik

(57) **Abstract**

Presented are concepts for providing subject-specific information. Once such concept is generating a subject-specific clinical model based on a plurality of clinical maps, clinical data relating to the subject and clinical standards information, wherein the subject specific clinical model comprises a hierarchical representation of the plurality of clinical maps.

## Description

### FIELD OF THE INVENTION

This invention relates to the field of subject information provision and more particularly to the visual provision of subject-specific information.

### BACKGROUND OF THE INVENTION

Known systems for providing subject information (such as clinical data) use clinical models and maps that are hard-coded and static.

A clinical map typically comprises graphical information for graphically representing clinical data relating to a subject. A clinical map may therefore provide a graphical representation of a region of anatomy or pathology of a human or animal. For instance, a clinical model may provide information for graphically representing clinical data relating to a heart, but may also be structured in a hierarchical form so that a refinement (e.g. sub-level) of the clinical model of the heart comprises a clinical model of a heart valve of the heart (e.g. information for graphically representing clinical data relating to the heart valve).

However, such conventional clinical models are not flexible enough to offer differently configured clinical models (e.g. because they are static). For instance, the provision of subject information (such a clinical data) may be subject to local standards and/or requirements. With clinical applications employing static, hard-coded clinical models, it can be impossible or very difficult to provide subject information according to local standards.

Also, where there may be multiple interacting clinical issues, conventional systems are unable to automatically provide or display clinical information from multiple clinical models in context (or in a manner which is quick and easy to interpret). With intuitive navigation of subject-specific clinical information according to local standards being of paramount importance in Clinical Decision Support (CDS), there remains a need for an approach to providing subject-specific information a manner which is flexible and intuitive.

### SUMMARY OF THE INVENTION

The invention aims to at least partly fulfil the aforementioned needs. To this end, the invention provides devices, systems and methods as defined in the independent claims. The dependent claims provide advantageous embodiments.

There is provided a method for providing subject-specific information. The method comprises: obtaining a plurality of clinical maps associated with a subject, each clinical map comprising graphical information for graphically representing clinical data relating to the subject; obtaining clinical data relating to the subject; obtaining clinical standards information relating to one or more local requirements for clinical data; and generating a subject-specific clinical model based on the plurality of clinical maps, the obtained clinical data and the obtained clinical standards information, the subject specific clinical model comprising a hierarchical representation of the plurality of clinical maps.

Proposed is a concept of composing a subject-specific clinical model from multiple clinical maps by combining (e.g. blending or merging) the clinical maps in consideration of available clinical data/information for the subject and local/applicable clinical requirements/constraints. In particular, combination of the clinical maps may be controlled based on: (i) available clinical subject information; (ii) one or more hierarchical clinical models; and (iii) a hierarchical organization of clinical maps. Such proposals may increase the clinical value of information by enabling clinical information to be visualised (e.g. displayed or graphically represented) relative to each other and in context. This may support assessment, enable improved focus on a diagnosis, and/or support clinical planning. Improved CDS may therefore be provided by proposed concepts. Also, proposed embodiments may support clinical information from multiple clinical models and enable intuitive visual navigation through subject-specific clinical (e.g. diagnostic) information according to local standards (e.g. encoded in a hierarchy adhering to local clinical models).

Proposed embodiments may provide a clinical model comprising structured health domain content that is specific to a subject. The provided clinical model may be structured in a hierarchy, thus expressing refinements of models according to clinical semantics (e.g. a heart valve model might be a refinement of a heart model). Such a clinical model may be generated from clinical maps, wherein a clinical map may be thought of a graphical interaction paradigm with structured clinical data. For instance, a clinical map may comprise a graphical representation of clinical data, or a definition thereof.

Accordingly, concepts are proposed which may enrich the display or visualisation of clinical information relating to a specific subject by creating a subject-specific clinical model using a plurality of clinical maps. The clinical maps may be combined according to available clinical information for the subject and local clinical models or requirements.

A clinical map, as opposed to a clinical model, may provide or define a graphical representation of a clinical data (e.g. a problem with an anatomical feature of a subject, treatment method(s) for a subject, medical support for pathologies, etc.). Some proposed embodiments may therefore be thought of overlaying a standard clinical model (e.g. anatomical atlas or map representation of a person) with one or more graphical representations indicating unique pathologies, clinical problems or treatments relevant to a specific-subject. Thus, there may be provided a medical system that facilitates interaction with structured or annotated aggregated clinical data relating to a specific subject.

Advantages associated with proposed embodiments may therefore include: (a) localization of clinical information representations without software engineering efforts; (b) intuitive navigation through clinical information; (c) increased clinical value of available information though the visualisation of clinical information elements relative to each other and/or in context (e.g. supporting assessment, focussing on a diagnosis, supporting planning, etc.).

There may be proposed a concept which takes: a plurality of clinical maps; a generic clinical model hierarchy according to local standards; and clinical data relating to a subject, and then uses that to generate a subject-specific clinical model, which provides a hierarchical representation of the clinical maps.

The step of generating a subject-specific clinical model may comprise: processing the obtained clinical data based on the obtained clinical standards information to generate converted clinical data adhering to the one or more local requirements; and processing each of the plurality of clinical maps based on the converted clinical data so as to generate a plurality of converted clinical maps, each converted clinical map comprising graphical information for graphically representing converted clinical data relating to the subject. A subject-specific clinical model comprising a hierarchical representation of the plurality of converted clinical maps may then be generated. Such embodiments may enable the provision of clinical maps adhering to local standards. For instance, a clinical model generated by an embodiment may be encoded with a hierarchy that matches (or conforms to) a localised clinical model. Put another way, a clinical model can be localized and associated with a plurality of clinical maps. Such an approach may provide subject-specific clinical maps, and such clinical maps may accord to a visualization or detail level according to subject specific information.

The subject-specific clinical model may be representative of an anatomical relationship between anatomical features of the subject. This may support the presentation and/or navigation of subject-specific clinical information in a manner which is representative a relative location and/or relationship in a subject's body. Simple and intuitive presentation or navigation of clinical information may thus be facilitated by proposed embodiments. For instance, by positioning clinical maps in a graphical representation of a subject's body according to the location of the associated anatomical feature(s), a clinical model may be provided which enables quick and easy navigation of the clinical maps.

The obtained clinical standards information may comprise information relating to one or more local requirements for presenting or navigating clinical data. The step of generating a subject-specific clinical model may then comprise defining a hierarchical representation of the plurality of clinical maps based on the information relating to one or more local requirements for presenting or navigating clinical data. In this way, embodiments may take account of local standards and/or requirements for clinical data. These standards/requirements may, for example, relate to the presentation, format, type and structure of clinical data.

The step of generating a subject-specific clinical model may comprise: combining at least two of the plurality of clinical maps based on a blending function. By way of example, the blending function may be representative of at least one of: a definition of an instance of a clinical model; a hierarchy of clinical models; and a hierarchy of clinical maps. A clinical model may thus be amended with multiple clinical maps, and such clinical maps may be combined into the model in a hierarchical manner and/or a manner which is representative of an anatomical relationship or context. Here, it is noted that a clinical map be defined as a part of another clinical map (e.g. a lower-level abstraction or sub-portion). A clinical map may therefore abstract another clinical map. Where an abstract representation is superimposed, a blending function may be adapted to avoid anatomical inconsistencies and adhere to a required rendering size.

Embodiments may further comprise annotating the generated subject-specific clinical model based on the obtained clinical standards information. For instance, one or more graphical representations may overlay a clinical map in a graphical representation of generated model. This may facilitate quick, easy and intuitive navigation of the clinical model.

According to another aspect of the invention, there is provided a method for displaying subject-specific information. The method comprises: providing subject-specific information according to a proposed embodiment; receiving an input signal representative of a selected clinical map of the subject-specific clinical model; generating a control signal for displaying clinical data relating to the subject based on the graphical information of the selected clinical map; and displaying the clinical data relating to the subject in accordance with the generated control signal.

According to another aspect of the invention, there is provided a system for providing subject-specific information. The system comprises: an input interface adapted to obtaining a plurality of clinical maps associated with a subject, each clinical map comprising graphical information for graphically representing clinical data relating to the subject, to obtain clinical data relating to the subject, and to obtain clinical standards information relating to one or more local requirements for clinical data; and a processing module adapted to generate a subject-specific clinical model based on the plurality of clinical maps, the obtained clinical data and the obtained clinical standards information, the subject specific clinical model comprising a hierarchical representation of the plurality of clinical maps.

The processing module may be adapted to: process the obtained clinical data based on the obtained clinical standards information to generate converted clinical data adhering to the one or more local requirements; process each of the plurality of clinical maps based on the converted clinical data so as to generate a plurality of converted clinical maps, each converted clinical map comprising graphical information for graphically representing converted clinical data relating to the subject; and generate a subject-specific clinical model comprising a hierarchical representation of the plurality of converted clinical maps.

In some embodiments, the obtained clinical standards information may comprise information relating to one or more local requirements for presenting or navigating clinical data. The processing module may then be adapted to define a hierarchical representation of the plurality of clinical maps based on the information relating to one or more local requirements for presenting or navigating clinical data.

In an embodiment, the processing module may be adapted to: combine at least two of the plurality of clinical maps based on a blending function. The blending function may be representative of at least one of: a definition of an instance of a clinical model; a hierarchy of clinical models; and a hierarchy of clinical maps.

According to yet another aspect of the invention, there is provided a system for displaying subject-specific information. The system comprises: a system for providing subject-specific information according to a proposed embodiment; a signal interface adapted to receive an input signal representative of a selected clinical map of the subject-specific clinical model; an output interface adapted to generate a control signal for displaying clinical data relating to the subject based on the graphical information of the selected clinical map; and a display unit adapted to display the clinical data relating to the subject in accordance with the generated control signal.

The processing module may be remotely located from the display unit, and the control signal may thus be communicated to the display unit via a communication link. In this way, a user (such as a medical professional) may have an appropriately arranged display system that can receive and display subject-specific information at a location remotely located from the processing module. Embodiments may therefore enable a user to remotely review subject-specific information using a portable display device, such as a laptop, tablet computer, mobile phone, PDA, etc.

The system may further comprise: a server device comprising the data processing module; and a client device comprising the display unit. Dedicated data processing means may therefore be employed for the purpose of generating a subject-specific clinical model and generating a control signal, thus reducing processing requirements or capabilities of other components or devices of the system.

The system may further comprise a client device, wherein the client device comprises the data processing module and the display unit. In other words, a user (such as a doctor or medical professional) may have an appropriately arranged client device (such as a laptop, tablet computer, mobile phone, PDA, etc.) which processes received data in order to generate a subject-specific clinical model and generate a control signal.

Thus, it will be understood that processing capabilities may therefore be distributed throughout the system in different ways according to predetermined constraints and/or availability of processing resources.

These and other aspects of the invention will be apparent from and elucidated with reference to the embodiment(s) described hereinafter.

### BRIEF DESCRIPTION OF THE DRAWINGS

Examples in accordance with aspects of the invention will now be described in detail with reference to the accompanying schematic drawings, in which:
Figure 1 is an exemplary flow diagram of a method for providing subject-specific information according to an embodiment;
Figure 2 is an exemplary flow diagram of a method for displaying subject-specific information according to an embodiment;
Figure 3 is an illustration summarising the hierarchical structure of a clinical model formed by combining a plurality of clinical maps in accordance with proposed concepts;
Figure 4 is a simplified block diagram of a system according to an embodiment; and
Figure 5 is a simplified block diagram of a computer within which one or more parts of an embodiment may be employed.

### DETAILED DESCRIPTION OF THE EMBODIMENTS

Proposed is a concept for presenting subject-specific clinical information in a visual form using clinical data maps. Clinical data maps may be combined into a subject-specific clinical model taking account of clinical data/information relating to the subject and local clinical standards/requirements. A clinical model provided by such proposals may thus facilitate simple and intuitive navigation and assessment of subject-specific clinical information, for example by enabling clinical information to be visualized and/or navigated in one or more contexts. Improved CDS may therefore be provided by proposed embodiments.

Embodiments of the present invention are therefore directed toward enabling the presentation of subject-specific clinical information so at to facilitate or enhance a CDS process. Further, embodiments may be aimed at enabling the display or visualisation of subject-specific clinical data or information with simplicity, accuracy and context while providing enough detail so that that a clinical assessment or decision is facilitated or supported (preferably with increased efficiency and/or reliability). A generated clinical model may be used to identify or indicate that a subject requires medical intervention, for example.

Embodiments are based on the insight that the display of clinical information may be enhanced by creating a subject-specific clinical model using a plurality of clinical maps that are combined according to subject-specific clinical information and local clinical standard/requirements (as represented by a local clinical model for example).

A clinical map, as opposed to a clinical model, may provide or define a graphical representation of a clinical data (e.g. a problem with an anatomical feature of a subject, treatment method(s) for a subject, medical support for pathologies, etc.).

A clinical model, on the other hand, may comprise structured health domain content. Thus, a clinical model may be structured in a hierarchy, thereby expressing refinements of models according to clinical semantics (e.g. a heart valve model might be a refinement of a heart model). Such a clinical model may be represented in the form of an anatomical atlas or map representation of a person, thus enabling visualisation of the anatomical features and available clinical data relating an anatomical feature.

According to proposed concepts, a clinical model may comprise clinical maps, wherein a clinical map may be thought of as a flexible (localized) graphical interaction paradigm with subject-specific clinical data.

A clinical (image) map may be a graphical representation of clinical data, and it is typically combined with a description of areas on the image (e.g. in an 'overlay' or multiple overlays). A clinical map can, for example, be 2D, 3D or even ND, where N is an integer greater than 3 (e.g. a movie of the heart contracting combined with another clinical map). The areas are then linked to elements described in a clinical model. A clinical model may therefore be thought of as a formal representation of entities in a domain and their relations (e.g. abstractions that describe aspects of a muscular system or digestive system). Thus, a clinical model may be structured in a hierarchy, thereby expressing refinements of models according to clinical semantics (e.g. a heart valve model might be a refinement of a heart model). Such a clinical model may be represented in the form of an anatomical atlas or map representation of a person, thus enabling visualisation of the anatomical features and available clinical data relating an anatomical feature. An example of a simple clinical model might be a clinical condition where the model might contain two attributes: an anatomical location and a severity.

The anatomical locations might be associated with or 'bound' to a value set (e.g. a set of anatomical descriptions), where the individual elements might be linked to an area in the map. A clinical model may thus contain all elements from the set, and an instance of the model may then contain only a subset of the elements (e.g. a particular location).

In this regard, it is noted that areas in a clinical map need not be restricted to being linked to elements in a value set. They might also be linked to other levels of aggregation such as models itself (e.g. types of abnormalities)

A (local or even subject specific) sketch of a clinical map, when associated with a clinical model (and as such 'inserted in the map hierarchy') can become part of the composed subject-specific clinical map according to a local clinical model.

Some proposed embodiments may therefore be thought of as combining clinical maps into a clinical model with one or more graphical representations indicating unique pathologies, clinical problems or treatments relevant to a specific-subject. Thus, there may be provided a medical system that facilitates interaction with structured or annotated aggregated clinical data relating to a specific subject. A clinical map may be provided which can support professionals, whilst other clinical maps may be adapted to support non-professional (e.g. a subject). Proposed concepts may thus be employed to compose: (i) a clinical map using professional clinical maps; (ii) a non-professional clinical map. Synchronization between clinical maps may also be enabled so as to facilitate the translation between domains (e.g. subject-specific domain and a localized domain employing local jargon or standards).

Illustrative embodiments may be utilized in many different types of clinical, medical or subject-related environments, such as a hospital, doctor's office, ward, care home, person's home, etc. In order to provide a context for the description of elements and functionality of the illustrative embodiments, the Figures are provided hereafter as examples of how aspects of the illustrative embodiments may be implemented. It should therefore be appreciated the Figures are only examples and are not intended to assert or imply any limitation with regard to the environments, systems or methods in which aspects or embodiments of the present invention may be implemented.

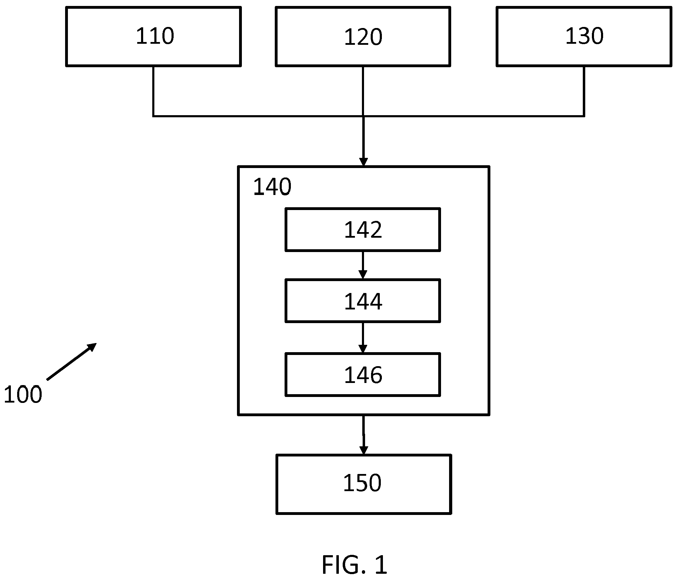

Referring now to Figure 1, there is depicted an exemplary flow diagram of a method 100 for providing subject-specific information according to an embodiment.

In step 110, a plurality of clinical maps associated with a subject is obtained. Each clinical map comprises graphical information for graphically representing clinical data relating to the subject. An example of a clinical map might be a professional 2-D image of coronary segments according to a specific classification system.

In step 120, clinical data relating to the subject is obtained. For example, specific clinical data might relate to a problem in the 7th segment of the Left Coronary Artery.

Clinical standards information relating to one or more local requirements for clinical data is obtained in step 130. For instance, a code for the Mid-Left Anterior Descending Coronary Artery according to the SNOMED standard might be 91748002. A clinical map specific for a subject might be a 3-D anatomical picture with the specific blood vessel highlighted until the location of the problem. This may require: a subject information model associated with multiple clinical maps and a list of all locations codified; clinical maps codified according to a standard; and subject data according to a clinical model.

Based on the plurality of clinical maps, the obtained clinical data and the obtained clinical standards information, a subject-specific clinical model is generated in step 140. Here, the generated subject-specific clinical model comprises a hierarchical representation of the plurality of clinical maps. Further, the subject-specific clinical model is representative of an anatomical relationship between anatomical features of the subject.

By way of example, the step 140 of generating the subject-specific clinical model in the embodiment of Figure 1 comprises a plurality of steps 142 through 146. More specifically, step 142 comprises processing the obtained clinical data (from step 120) based on the obtained clinical standards information (from 130) to generate converted clinical data adhering to the one or more local requirements. Each of the plurality of clinical maps are then processed in step 144 based on the converted clinical data so as to generate a plurality of converted clinical maps, wherein each converted clinical map comprises graphical information for graphically representing converted clinical data relating to the subject. Then, in step 146, a subject-specific clinical model is generated, wherein the subject-specific clinical model comprises a hierarchical representation of the plurality of converted clinical maps.

Thus, it will be understood that a model provided by an embodiment may, for example, describe a heart in a generic manner with a value set of relevant vessels. Embodiments may enable a user should be able to insert their own clinical image according to their education in the system and codify it accordingly. The subject specific information can be superimposed on the image, which then becomes subject-specific. Also, the specific image might become part of a set of standard images, e.g. their vessel sketch may be superimposed on a standard anatomical image of the chest. Furthermore a specific model of the lesion might be linked to a graphical symbol summarizing the type of lesion on the image.

Also, a clinical map may itself be codified and as such, it may then be placed in context with other existing clinical maps.

By way of further example, in another embodiment of the method the obtained clinical standards information can comprise information relating to one or more local requirements for presenting or navigating clinical data. Based on such information relating to one or more local requirements for presenting or navigating clinical data, the step 140 of generating a subject-specific clinical model can include defining a hierarchical representation of the plurality of clinical maps. In this way, the hierarchical representation of the plurality of clinical maps provided by the generated model may adhered to the local requirement(s) standards.

In yet another example, the step 140 of generating a subject-specific clinical model can include combining at least two of the plurality of clinical maps based on a blending function. The blending function may be a function which is representative of at least one of: a definition of an instance of a clinical model; a hierarchy of clinical models; and a hierarchy of clinical maps.

Furthermore, as depicted in Figure 1, the proposed embodiment also includes the step 150 of annotating the generated subject-specific clinical model based on the obtained clinical standards information. For instance, one or more graphical representations may be overlaid on a clinical map in a graphical representation of generated model. This can facilitate quick, easy and intuitive navigation of the clinical model.

It will be appreciated that execution of the method of Figure 1 provide a subject-specific clinical model, and this may then be leveraged for the purpose of displaying subject-specific information to user (such as a medical professional for example). Proposed concepts may therefore provide a method for displaying subject specific information which incorporates an embodiment of providing subject-specific information.

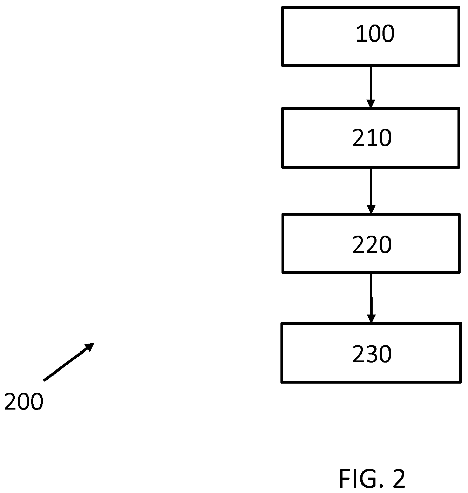

By way of example, Figure 2 depicts an exemplary flow diagram of a method 200 for displaying subject-specific information according to an embodiment, wherein the method 200 employs the method 100 of Figure 1.

More specifically, the method 200 begins with the step 100 of providing subject-specific information according to proposed embodiment of Figure 1. Execution of step 100 therefore results in the provision of a subject-specific clinical model based on a plurality of clinical maps, clinical data and clinical standards information.

In step 210, an input signal is received which is representative of a selected clinical map of the subject-specific clinical model (from step 100). A control signal is then generated in step 220 based on the graphical information of the selected clinical map, wherein the control signal is for controlling a display device to display clinical data relating to the subject.

By way of further example, however, the control signal can also be generated based on a display position of the selected clinical map of the subject-specific clinical model. In this way, display considerations (such available display space, already-displayed graphical elements, overlapping content, etc.) may be accounted for so as to ensure correct or appropriate display instructions are provided by the control signal.

The clinical data relating to the subject is then displayed in step 230 in accordance with the generated control signal.

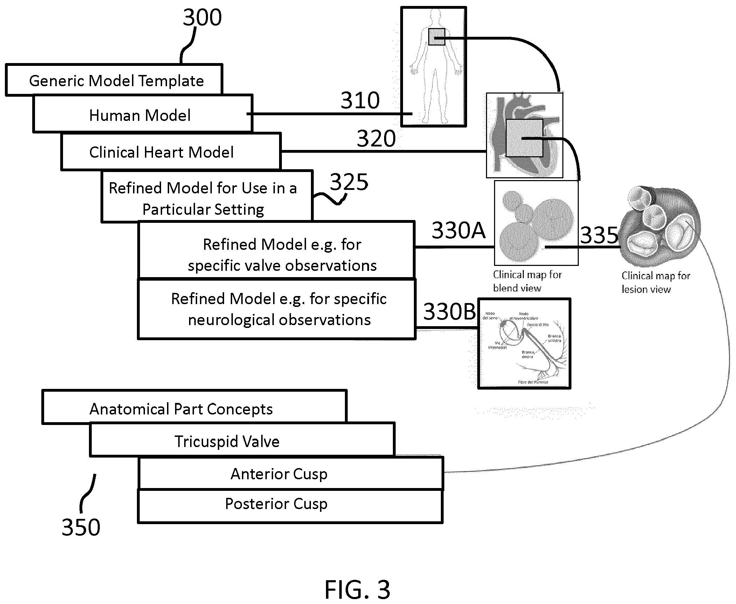

Referring now to Figure 3, there is provided an illustration summarising the hierarchical structure of a clinical model formed by combining a plurality of clinical maps in accordance with proposed concepts.

From a generic model template 300 there is defined a human model 310. As part of the model, there is defined a clinical heart model 320. A refined version 325 of the clinical heart model 320 can be defined for use in a particular setting. The refined model 325 in the example of Figure 3 comprises a first refined model 330A for specific heart valve observations, and a second refined model 330B for specific neurological observations. Here, the first refined model 330A comprises a clinical map 330A which further comprises a clinical map 335 for a lesion view of the valve observations. A clinical map 330A may thus abstract another clinical map 335. Representation of this hierarchy may be realised by superimposing graphical elements in the blending function so as to avoid anatomical inconsistencies.

It is also noted that the clinical map 335 for a lesion view of the valve observations can be linked to another (second) hierarchical model 350. In this way, a clinical map may be related to multiple models, and this may be cross-hierarchy (e.g. the clinical map may be a differing hierarchical levels in different models).

Thus, from Figure 3, it will be appreciated that a clinical model 310 may be amended with multiple clinical maps (e.g. 330A and 330B. Also, a hierarchy of the clinical maps can be defined and represented by the model. Furthermore, a region on a clinical map can reference to any other model, thus indicating a relationship between models (as illustrated by the clinical map 335 for a lesion view of the valve observations being linked to second hierarchical model 350).

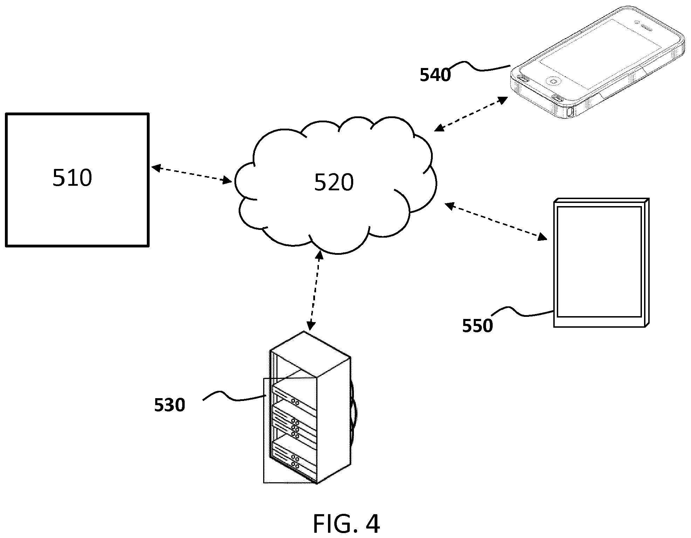

Referring now to Fig. 4, there is depicted an embodiment of a system according to an embodiment of the invention comprising an input system 510 arranged to obtain various sets of information

Here, the input system 510 is adapted to obtain a plurality of clinical maps associated with a subject, each clinical map comprising graphical information for graphically representing clinical data relating to the subject, to obtain clinical data relating to the subject, and to obtain clinical standards information relating to one or more local requirements for clinical data. The input system 510 is adapted to output one or more signals which are representative of obtained information/data.

The input system 510 communicates the output signals via the internet 520 (using a wired or wireless connection for example) to a remotely located data processing system 530 (such as server).

The data processing system 530 is adapted to receive the one or more output signals from the input system 510 and process the received signal(s) to generate a subject-specific clinical model, wherein the subject-specific clinical model comprising a hierarchical representation of a plurality of clinical maps. Thus, the data processing 530 provides a centrally accessible processing resource that can receive information from the input system 510 and run one or more algorithms to transform the received information into a subject-specific clinical model. Information relating to the subject-specific clinical model can be stored by the data processing system (for example, in a database) and provided to other components of the monitoring system. Such provision of information about a subject-specific clinical model may be undertaken in response to a receiving a request (via the internet 520 for example) and/or may be undertaken without request (i.e. 'pushed').

For the purpose of receiving information about a subject-specific clinical model from the data processing system 530, and thus to subject-specific information to be viewed, the system further comprises first 540 and second 550 mobile computing devices.

Here, the first mobile computing device 540 is a mobile telephone device (such as a smartphone) with a display for displaying clinical maps in accordance with embodiments of the proposed concepts. The second mobile computing device 550 is a mobile computer such as a Laptop or Tablet computer with a display for displaying clinical maps in accordance with embodiments of the proposed concepts.

The data processing system 530 is adapted to communicate clinical model output signals to the first 540 and second 550 mobile computing devices via the internet 520 (using a wired or wireless connection for example). As mentioned above, this may be undertaken in response to receiving a request from the first 540 or second 550 mobile computing devices.

Based on the received output signals, the first 540 and second 550 mobile computing devices are adapted to display one or more graphical elements in a display area provided by their respective display. For this purpose, the first 540 and second 550 mobile computing devices each comprise a software application for processing, decrypting and/or interpreting received clinical model output signals in order to determine how to display graphical elements. Thus, the first 540 and second 550 mobile computing devices each comprise a processing arrangement adapted to determine an attribute of clinical model and/or clinical map, and to generate a display control signal for modifying at least one of the size, shape, position, orientation, pulsation or colour of a graphical element based on the determined attribute of the clinical model and/or clinical map.

The system can therefore communicate information about subject-specific clinical models to users of the first 540 and second 550 mobile computing devices. For example, each of the first 540 and second 550 mobile computing devices may be used to display graphical elements to a medical practitioner, doctor, consultant, technician or caregiver for example.

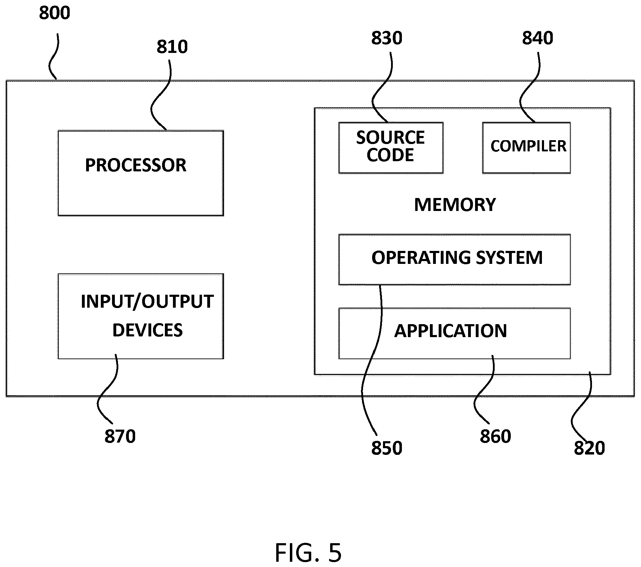

Implementations of the system of Figure 5 may vary between: (i) a situation where the data processing system 530 communicates display-ready clinical model data, which may for example comprise display data including graphical elements (e.g. in JPEG or other image formats) that are simply displayed to a user of a mobile computing device using conventional image or webpage display (can be web based browser etc.); to (ii) a situation where the data processing system 530 communicates raw data set information that the receiving mobile computing device then transforms to a clinical model, processes to determine one or more attributes of the clinical map, and then displays graphical elements based on the determined one or more attributes (for example, using local software running on the mobile computing device). Of course, in other implementations, the processing may be shared between the data processing system 530 and a receiving mobile computing device such that part of the clinical model generated at data processing system 530 is sent to the mobile computing device for further processing by local dedicated software of the mobile computing device. Embodiments may therefore employ server-side processing, client-side processing, or any combination thereof.

Further, where the data processing system 530 does not 'push' subject-specific clinical model information (e.g. clinical model output signals), but rather communicates information in response to receiving a request, the user of a device making such a request may be required to confirm or authenticate their identity and/or security credentials in order for information to be communicated.

Figure 5 illustrates an example of a computer 800 within which one or more parts of an embodiment may be employed. Various operations discussed above may utilize the capabilities of the computer 800. For example, one or more parts of a system for providing subject-specific information (or display unit thereof) may be incorporated in any element, module, application, and/or component discussed herein.

The computer 800 includes, but is not limited to, PCs, workstations, laptops, PDAs, palm devices, servers, storages, and the like. Generally, in terms of hardware architecture, the computer 800 may include one or more processors 810, memory 820, and one or more I/O devices 870 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 810 is a hardware device for executing software that can be stored in the memory 820. The processor 810 can be virtually any custom made or commercially available processor, a central processing unit (CPU), a digital signal processor (DSP), or an auxiliary processor among several processors associated with the computer 800, and the processor 810 may be a semiconductor based microprocessor (in the form of a microchip) or a microprocessor.

The memory 820 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 820 may incorporate electronic, magnetic, optical, and/or other types of storage media. Note that the memory 820 can have a distributed architecture, where various components are situated remote from one another, but can be accessed by the processor 810.

The software in the memory 820 may include one or more separate programs, each of which comprises an ordered listing of executable instructions for implementing logical functions. The software in the memory 820 includes a suitable operating system (O/S) 850, compiler 840, source code 830, and one or more applications 860 in accordance with exemplary embodiments. As illustrated, the application 860 comprises numerous functional components for implementing the features and operations of the exemplary embodiments. The application 860 of the computer 800 may represent various applications, computational units, logic, functional units, processes, operations, virtual entities, and/or modules in accordance with exemplary embodiments, but the application 860 is not meant to be a limitation.

The operating system 850 controls the execution of other computer programs, and provides scheduling, input-output control, file and data management, memory management, and communication control and related services. It is contemplated by the inventors that the application 860 for implementing exemplary embodiments may be applicable on all commercially available operating systems.

Application 860 may be a source program, executable program (object code), script, or any other entity comprising a set of instructions to be performed. When a source program, then the program is usually translated via a compiler (such as the compiler 840), assembler, interpreter, or the like, which may or may not be included within the memory 820, so as to operate properly in connection with the O/S 850. Furthermore, the application 860 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 870 may include input devices such as, for example but not limited to, a mouse, keyboard, scanner, microphone, camera, etc. Furthermore, the I/O devices 870 may also include output devices, for example but not limited to a printer, display, etc. Finally, the I/O devices 870 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 870 also include components for communicating over various networks, such as the Internet or intranet.

If the computer 800 is a PC, workstation, intelligent device or the like, the software in the memory 820 may further include a basic input output system (BIOS) (omitted for simplicity). The BIOS is a set of essential software routines that initialize and test hardware at startup, start the O/S 850, and support the transfer of data among the hardware devices. The BIOS is stored in some type of read-only-memory, such as ROM, PROM, EPROM, EEPROM or the like, so that the BIOS can be executed when the computer 800 is activated.

When the computer 800 is in operation, the processor 810 is configured to execute software stored within the memory 820, to communicate data to and from the memory 820, and to generally control operations of the computer 800 pursuant to the software. The application 860 and the O/S 850 are read, in whole or in part, by the processor 810, perhaps buffered within the processor 810, and then executed.

When the application 860 is implemented in software it should be noted that the application 860 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 860 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, statesetting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand various embodiments with various modifications are contemplated.

## Claims

1. A method for providing subject-specific information, the method comprising:
obtaining a plurality of clinical maps associated with a subject, each clinical map comprising graphical information for graphically representing clinical data relating to the subject;
obtaining clinical data relating to the subject;
obtaining clinical standards information relating to one or more local requirements for clinical data; and
generating a subject-specific clinical model based on the plurality of clinical maps, the obtained clinical data and the obtained clinical standards information, the subject specific clinical model comprising a hierarchical representation of the plurality of clinical maps.

2. The method of claim 1, wherein the step of generating a subject-specific clinical model comprises:
processing the obtained clinical data based on the obtained clinical standards information to generate converted clinical data adhering to the one or more local requirements;
processing each of the plurality of clinical maps based on the converted clinical data so as to generate a plurality of converted clinical maps, each converted clinical map comprising graphical information for graphically representing converted clinical data relating to the subject; and
generating a subject-specific clinical model comprising a hierarchical representation of the plurality of converted clinical maps.

3. The method of claim 1 or 2, wherein the subject-specific clinical model is representative of an anatomical relationship between anatomical features of the subject.

4. The method of any preceding claim, wherein the obtained clinical standards information comprises information relating to one or more local requirements for presenting or navigating clinical data,
and wherein the step of generating a subject-specific clinical model comprises defining a hierarchical representation of the plurality of clinical maps based on the information relating to one or more local requirements for presenting or navigating clinical data.

5. The method of any preceding claim, wherein the step of generating a subject-specific clinical model comprises:
combining at least two of the plurality of clinical maps based on a blending function,
and wherein the blending function is representative of at least one of: a definition of an instance of a clinical model; a hierarchy of clinical models; and a hierarchy of clinical maps.

6. The method of any preceding claim, further comprising:
annotating the generated subject-specific clinical model based on the obtained clinical standards information.

7. A method for displaying subject-specific information, the method comprising:
providing subject-specific information according to any preceding claim;
receiving an input signal representative of a selected clinical map of the subject-specific clinical model;
generating a control signal for displaying clinical data relating to the subject based on the graphical information of the selected clinical map; and
displaying the clinical data relating to the subject in accordance with the generated control signal.

8. The method of claim 7, wherein the step of generating a control signal is further based on a display position of the selected clinical map of the subject-specific clinical model.

9. A computer program product for providing subject-specific information, wherein the computer program product comprises a computer-readable storage medium having computer-readable program code embodied therewith, the computer-readable program code configured to perform all of the steps of any preceding claim.

10. A computer system comprising: a computer program product according to claim 9; and one or more processors adapted to perform a method according to any of claims 1 to 8 by execution of the computer-readable program code of said computer program product.

11. A system for providing subject-specific information, the system comprising:
an input interface adapted to obtaining a plurality of clinical maps associated with a subject, each clinical map comprising graphical information for graphically representing clinical data relating to the subject, to obtain clinical data relating to the subject, and to obtain clinical standards information relating to one or more local requirements for clinical data; and
a processing module adapted to generate a subject-specific clinical model based on the plurality of clinical maps, the obtained clinical data and the obtained clinical standards information, the subject specific clinical model comprising a hierarchical representation of the plurality of clinical maps.

12. The system of claim 11, wherein the processing module is adapted to:
process the obtained clinical data based on the obtained clinical standards information to generate converted clinical data adhering to the one or more local requirements;
process each of the plurality of clinical maps based on the converted clinical data so as to generate a plurality of converted clinical maps, each converted clinical map comprising graphical information for graphically representing converted clinical data relating to the subject; and
generate a subject-specific clinical model comprising a hierarchical representation of the plurality of converted clinical maps.

13. The system of any of claims 10 to 12, wherein the obtained clinical standards information comprises information relating to one or more local requirements for presenting or navigating clinical data,
and wherein the processing module is adapted to define a hierarchical representation of the plurality of clinical maps based on the information relating to one or more local requirements for presenting or navigating clinical data.

14. The system of any of claims 10 to 13, wherein the processing module is adapted to:
combine at least two of the plurality of clinical maps based on a blending function,
and wherein the blending function is representative of at least one of: a definition of an instance of a clinical model; a hierarchy of clinical models; and a hierarchy of clinical maps.

15. A system for displaying subject-specific information, the system comprising:
a system for providing subject-specific information according to any of claims 10 to 14;
a signal interface adapted to receive an input signal representative of a selected clinical map of the subject-specific clinical model;
an output interface adapted to generate a control signal for displaying clinical data relating to the subject based on the graphical information of the selected clinical map; and
a display unit adapted to display the clinical data relating to the subject in accordance with the generated control signal.
